# EUROPEAN PATENT SPECIFICATION

(11) **EP 4 134 058 B1**
(45) Date of publication and mention of the grant of the patent: **22.01.2025**
(21) Application number: 21873821.9
(22) Date of filing: 19.03.2021
(51) Int. Cl.: A61H 1/02, B25J 9/00, A61F 5/00, F16D 51/20

(54) **EXOSKELETON JOINT SELF-LOCKING MECHANISM, KNEE JOINT, AND BIONIC REHABILITATION ROBOT**
EXOSKELETTGELENKSELBSTSPERRMECHANISMUS, KNIEGELENK UND BIONISCHER REHABILITATIONSROBOTER
MÉCANISME AUTOBLOQUANT D'ARTICULATION D'EXOSQUELETTE, ARTICULATION DE GENOU ET ROBOT DE RÉÉDUCATION BIONIQUE

(30) Priority: 29.09.2020 CN 202011046110
(43) Date of publication of application: 15.02.2023
(73) Proprietor: SHANGHAI FOURIER INTELLIGENCE CO., LTD, Shanghai 201203 (CN)
(72) Inventor: WANG, Jun, Huaiyin District Huaian, Jiangsu 223000 (CN); WANG, Tongchen, Shanghai 200000 (CN); XIAO, Danping, Town Nankang, Jiangxi 341400 (CN); GU, Jie, Shanghai 200000 (CN); LI, Chong, Shanghai 200000 (CN)
(74) Representative: Chung, Hoi Kan
(86) International application number: PCT/CN2021/081710
(87) International publication number: WO 2022/068154

(56) References cited:
- EP-A1- 2 198 810
- WO-A1-2014/039134
- WO-A1-2018/236225
- CN-A- 1 286 187
- CN-A- 103 260 576
- CN-A- 106 321 690
- CN-A- 107 928 995
- CN-A- 108 742 967
- CN-A- 111 920 651
- CN-U- 206 630 847
- CN-U- 210 889 836
- CN-Y- 2 203 726

## Description

### FIELD OF TECHNOLOGY

The present invention relates to the technical field of medical rehabilitation devices, in particular to an exoskeleton joint self-locking mechanism, a knee joint and a bionic rehabilitation robot.

### BACKGROUND

With the continuous development of medical science and technology, rehabilitation therapy has become a new therapeutic discipline to promote the rehabilitation of physical and mental functions of patients and disabled people, and it is also a new technical specialty. Its purpose is to enable people to resume their daily life, study, work and labor, as well as social life as possible, integrate into society and improve their quality of life.

Patients with limb loss of mobility and muscle atrophy due to cerebrovascular diseases, brain trauma and other causes often rely on external forces to assist them in standing and walking for rehabilitation treatment. Therefore, rehabilitation robot technologies such as walking aids have been widely developed in the world, such as:
① Knee joint structure with self-locking function: "Hebei University of Technology", "A wearable lower limb walking aid exoskeleton imitating human knee joint movement curve", patent grant announcement number CN 210872826 U:
   For the knee joint of this lower limb aid exoskeleton, after the thigh motor is started, the rotation is transmitted to the thigh torque sensor through the first connecting flange of the thigh, the thigh torque sensor drives the second connecting flange of the thigh to rotate, and the output end of the second connecting flange of the thigh transmits power to the lead screw; through the back and forth rotation of the lead screw, the slide on the lead screw is driven to move up and down, so that the thigh auxiliary support drives the shank main support to expand and contract, thus realizing the rotation of the knee joint. Although the knee joint with this structure has a self-locking function, it has large weight and size. As far as the power of the motor selected for the knee joint, energy consumption is large, and the electrified endurance time of the whole machine is short.
② "MEBOTX INTELLIGENT TECH SUZHOU CO., LTD.", "A lower limb load-bearing assisting exoskeleton capable of achieving rapid self-locking in standing state", patent application publication number CN 111409060 A:
   The locking mechanism in this patent comprises a driver, a node box, a fixing seat, a spring, a locking pin, a manual switch assembly, a path cover, a locking handle, a cable sleeve upper joint, a cable steel rope, a cable sleeve and a locking hole.

When the joint needs to be locked, the wearer presses the switch located elsewhere, and the main control device will send a signal to the node box, and the node box controls an end round bar of the driver to push out upward, after the end round bar is pushed out upwards, the locking pin can be pushed out; at this time, the spring is compressed, and the end of the locking pin is inserted into the locking hole. After the activation state of the locking unit is completed, the driver can be powered off. Due to the internal screw structure, the round bar at the end of the driver can realize linear self-locking, that is, the locking pin cannot be retracted. In addition, the wearer can manually pull the handle to drive the cable steel rope to pull the top post at the lower end of the steel rope to push out the locking pin to realize locking.

This knee joint structure can only be locked and unlocked by manual or remote control. The locking and unlocking of the joint of a patient during walking require frequent human control intervention, without automatic and intelligent control. The complicated operation can easily lead to manipulation errors, which can't protect the safety of the wearer. Moreover, the user experience is poor, and it is impossible to realize the high bionics of the human walking state.

③ "University of California" "A controllable passive artificial knee", patent application publication number CN 104822346 A:
In this patent, one end of a torsion spring is sleeved on a disk body, the other end is sleeved on a rotating shaft of a shank connector, and the radius of the torsion spring is slightly smaller than the disk body. The disk body is fixed on the cover body to ensure that the torsion spring does not rotate around the disk body. The leading end of the torsion spring passes through the hole in the rod of the brake. Therefore, when the brake controls the rod to stretch out and draw back, the leading end will also move, which will cause the torsion spring to be compressed or stretched, and press or loosen the rotating shaft. In this way, a resistance distance is generated between the thigh connector and the shank connector, thus realizing the function of locking and unlocking the knee j oint. In the example described in the patent, the brake is controlled by an angle sensor, which determines the working state of the brake by sensing the swing amplitude of the wearer's thigh and controls the unlocking and locking of the knee joint.

④ "CHONGQING ZHONGDI MACHINERY MFG CO LTD", "Aluminum alloy wedge-type brake assembly", patent application publication number CN 106321690 B:
This patent belongs to the field of automotive brakes, and particularly discloses an aluminum-alloy wedge-type brake assembly. The assembly has a friction piece fixed on inner and outer surfaces of a brake shoe. The brake shoe is fixed with a brake drum that is provided with a left brake shoe and a right brake shoe. The left brake shoe and the right brake shoe are hinged with a brake soleplate by a pin shaft. The left brake shoe and the right brake shoe are provided with a return elastic piece. An expander is provided with a wedge-shaped guide block. A wedge-shaped rod drives the wedge-shaped rod and a pneumatic mechanism and fixed with the guide block.

Given the shortcomings in the prior art, the present invention came into being.

### SUMMARY

The present invention provides an exoskeleton joint self-locking mechanism according to appended claim 1.

By adopting the technical solution, the present invention has the following technical effects:
According to the present invention, the first base and the second base are rotatably installed, and then the first base and the second base are fixed on the limb of the joint, thereby realizing flexible rotation at the joint; then, a mutual friction surface is formed between the rotating outward expanding locking member on the first base and the inner wall of the second base. In an initial state, the first rotating frame, the second rotating frame and the first base do not contact or generate self-locking friction force; when turning to a self-locking state, the locking driving member drives the rotating outward expanding locking member to expand outward, so that the friction surface is in contact and self-locked; the driving is progressive, so a magnitude of the friction force is also progressive; the knee joint is locked and unlocked by controlling the friction force so that the unlocking and locking of the knee joint are not completed instantaneously, and it is gradually locked and unlocked with the increase and decrease of friction force, which is more in line with the activity habits of the joint and safer.

Further, the free ends of the first rotating frame and/or the second rotating frame are provided with a stress slope surface, the locking driving member is provided with a telescopic locking portion, which has a force applying slope surface, and the force applying slope surface applies force to the stress slope surface along with the telescopic locking portion. During the sliding of the stress slope surface and the force applying slope surface against each other, the force applying slope surface applies a force to the stress slope surface to separate the first rotating frame and the second rotating frame.

Preferably, an inverted trapezoidal space is formed between the stress slope surfaces of the first rotating frame and the second rotating frame, and the shape of the telescopic locking portion matches with the inverted trapezoidal space. The inverted trapezoidal space formed by the first rotating frame and the second rotating frame can make the force more uniform.

Preferably, a driving end of the driving motor is provided with a threaded segment, the locking slider is provided with a threaded groove, and the threaded segment is inserted into the threaded groove. The driving motor is enabled to accurately adjust the rotation of the threaded segment through a reduction gearbox, thus accurately adjusting the advance and retreat of the locking slider, so that the whole self-locking process is smooth and natural.

Preferably, the second base is mounted to the first base through a mounting mechanism, and the mounting mechanism comprises:
a mounting groove provided on the second base;
a bearing, the outer ring of the bearing being fixedly mounted in the mounting groove; and
a rotating shaft disposed in the first compartment of the first base, and the inner ring of the bearing being fixedly mounted on the rotating shaft.

It facilitates the relative rotation of the first base and the second base without axial translocation, and the rotation is smooth and stable.

Preferably, the free ends of the first rotating frame and the second rotating frame are connected by an elastic return member.

The present invention also discloses an exoskeleton joint according to claim 6. The self-locking mechanism is disposed at the knee joint, which can start the locking driving member to complete the self-locking to assist standing when standing, and can unlock when walking to complete the activities at the knee joint.

The present invention also discloses an exoskeleton bionic rehabilitation robot according to claim 7. As mentioned above, the locking driving member can be started to complete self-locking to assist standing when standing, and the activities at the knee joint can be unlocked when walking.

Preferably, the exoskeleton bionic rehabilitation robot also includes a control unit, which comprises:
a control motherboard being in control connection to the locking driving member; and
a ranging sensor disposed on the ankle-foot component to measure the distance between the ankle-foot component and the walking surface, and the ranging sensor being in signal connection to the control motherboard.

The distance between the foot and the walking surface (ground) is measured to determine whether the leg walks or stands. When standing, the distance between the ranging sensor and the walking surface is the smallest, then self-locking is performed to assist standing; and when the distance between the ranging sensor and the walking surface becomes larger, it is in the state of walking or preparing to walk, and activities of the knee joint are completely by the progressive unlocking.

Preferably, the ranging sensor includes an infrared ranging sensor, a laser ranging sensor, an ultrasonic ranging sensor, and a radar ranging sensor. The range sensor is designed to measure the distance between the ankle-foot component and the walking surface to determine whether the leg is standing or lifting from the ground to prepare for walking.

According to the present invention, by synchronizing the driving shaft with the synchronous shaft, the measuring turntable also rotates, the number of times that the signal transmitter and signal receiver go through the signal marked area and the signal unmasked area till realizing the self-locking state is counted, so that an angle at which the measuring turntable has turned can be read and recorded, which can prevent the excessive rotation of the driving motor from damaging other parts, and can also control the time to reach this angle, so that the speed of self-locking can be controlled, and the bionic real knee joint movement can be improved for comfort and safety.

Preferably, the signal transmitter and the signal receiver are a set of infrared transceiver photodiode pairs.

Preferably, the measuring turntable is a raster encoder.

Preferably, the driving motor is a coaxial motor, and the synchronous shaft and the driving shaft of the driving motor are disposed to be coaxial but at different ends.

### BRIEF DESCRIPTION OF THE DRAWINGS

FIG. 1 is a structural schematic diagram of the exoskeleton joint self-locking mechanism of the present invention.
FIG. 2 is an exploded view of the exoskeleton joint self-locking mechanism of the present invention.
FIG. 3 is a structural diagram of the first base in the exoskeleton joint self-locking mechanism of the present invention.
FIG. 4 is a schematic diagram of the stress slope surface and the force applying slope surface in the exoskeleton joint self-locking mechanism of the present invention.
FIG. 5 is a structural diagram of the driving motor in the exoskeleton joint self-locking mechanism of the present invention.
FIG. 6 is a structural diagram of the measuring turntable in the exoskeleton joint self-locking mechanism of the present invention.
FIG. 7 is a sectional view of the driving motor in the exoskeleton joint self-locking mechanism of the present invention.
FIG. 8 is a structural diagram of the exoskeleton knee joint of the present invention.
FIG. 9 is a structural schematic diagram of the exoskeleton bionic rehabilitation robot of the present invention.
FIG. 10 is a structural schematic diagram of the ankle-foot component in the exoskeleton bionic rehabilitation robot of the present invention.
FIG. 11 is an assembly diagram of the ankle-foot component in the exoskeleton bionic rehabilitation robot of the present invention.
FIG. 12 is a whole sectional view of the exoskeleton bionic rehabilitation robot of the present invention.
FIG. 13 is a functional schematic diagram of the remote control of the exoskeleton bionic rehabilitation robot of the present invention.

In the drawings:

| | |
|---|---|
| first base | 1 |
| second base | 2 |
| angle limiting block | 11 |
| first rotating frame | 31 |
| stress slope surface | 311 |
| second rotating frame | 32 |
| brake pad | 33 |
| elastic return member | 34 |
| outer cover plate | 4 |
| rotating shaft | 51 |
| bearing | 52 |
| clamp spring | 53 |
| driving motor | 61 |
| miniature DC motor | 611 |
| reduction gearbox | 612 |
| mounting bearing | 613 |
| threaded segment | 614 |
| locking slider | 62 |
| force applying slope surface | 621 |
| raster encoder | 71 |
| signal masked area | 711 |
| signal unmasked area | 712 |
| infrared transceiver photodiode pairs | 72 |
| control motherboard | 73 |
| synchronous shaft | 74 |
| shank connecting rod | 8 |
| ankle-foot component | 9 |
| ranging sensor | 91 |
| ranging sensor bracket | 92 |
| third base | 93 |
| fourth base | 94 |
| ankle-foot rotating shaft | 95 |
| ankle-foot bearing | 96 |

### DETAILED DESCRIPTION OF THE EMBODIMENTS

Embodiments of the present invention are described below by way of specific examples, and other advantages and effects of the present invention will be readily apparent to those skilled in the art from the disclosure of this description. The present invention may also be practiced or applied by other different embodiments, and the details of this description may be modified or changed in various ways without departing from the essence of the present invention, based on different views and applications.

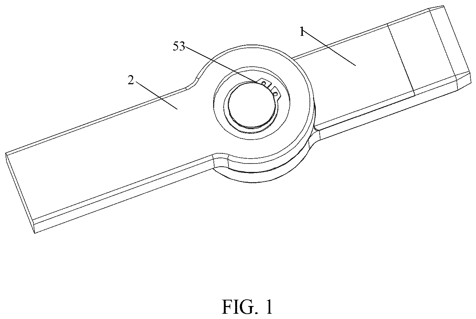

Referring to FIG. 1, FIG. 2 and FIG. 3, the present invention primarily provides an exoskeleton joint self-locking mechanism, which comprises a first base 1, a rotating outward expanding locking member, a second base 2 and a locking driving member, wherein the first base 1 has a first compartment; the rotating outward expanding locking member is disposed in the first compartment, the rotating outward expanding locking member comprises a first rotating frame 31 and a second rotating frame 32, and outer sides of the first rotating frame 31 and the second rotating frame 32 are provided with a first friction surface; one end of the first rotating frame 31 is pivoted with one end of the second rotating frame 32, and the other ends of the first rotating frame 31 and the second rotating frame are relatively free ends; the second base 2 has a second compartment; the second base 2 is rotatably mounted on the first base, and the inner wall of the second compartment encloses the rotating outward expanding locking member and forms a second friction surface matching the first friction surface; and the locking driving member can apply/cancel a force pushing away from the free end of the first rotating frame 31 and the free end of the second rotating frame 32, to make the first friction surface close contact to lock/unlock the second friction surface. According to the present invention, the first base 1 and the second base 2 are rotatably installed, and then the first base 1 and the second base 2 are fixed on the limb of the joint, thereby realizing flexible rotation at the joint; then, mutual friction surfaces are formed between the rotating outward expanding locking member on the first base 1 and the inner wall of the second base 2. In an initial state, the first rotating frame 31, the second rotating frame 32 and the first base do not contact or generate self-locking friction force; when turning to a self-locking state, the locking driving member drives the rotating outward expanding locking member to expand outward, so that the friction surfaces are in contact and self-locked; the driving is progressive, so a magnitude of the friction force is also progressive; the knee joint is locked and unlocked by controlling the friction force, so the unlocking and locking of the knee joint are not completed instantaneously, but gradually locked and unlocked with the increase and decrease of friction force, which is more in line with the activity habits of the joint and safer.

The following is described in detail by way of specific embodiments in conjunction with the accompanying drawings:
As for the pivoting of the first rotating frame 31 and the second rotating frame 32, the implementation structure in this embodiment is represented as the end of the first rotating frame 31 and the second rotating frame 32 are respectively provided with a lug, two lugs are provided with holes, and the two lugs are stacked together and then inserted and mounted by a pivot mounting shaft.

The design at the joint should favor the space utilization, make the overall component space layout reasonable and compact, and reduce the overall size so that the force applied by the locking driving member is divided and decomposed by two acting surfaces. Specifically, the free ends of the first rotating frame 31 and/or the second rotating frame 32 are provided with a stress slope surface, the locking driving member is provided with a telescopic locking portion, the telescopic locking portion has a force applying slope surface 621, and the force applying slope surface 621 applies force to the stress slope surface along with the telescopic locking portion. In this way, when the stress slope surface and the force applying slope surface 621 slide against each other, the force applying slope surface 621 applies a force to the stress slope surface to separate the first rotating frame 31 from the second rotating frame 32, so that the overall structure is flattened, which is more suitable for wearing and mounting at the joint.

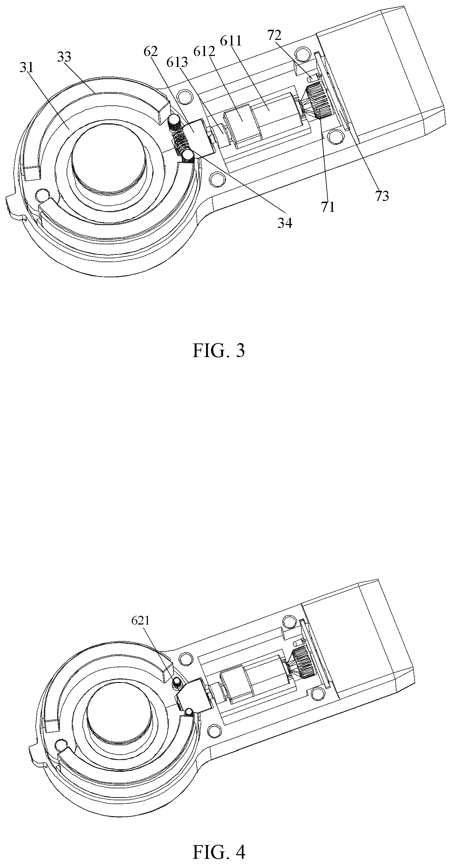

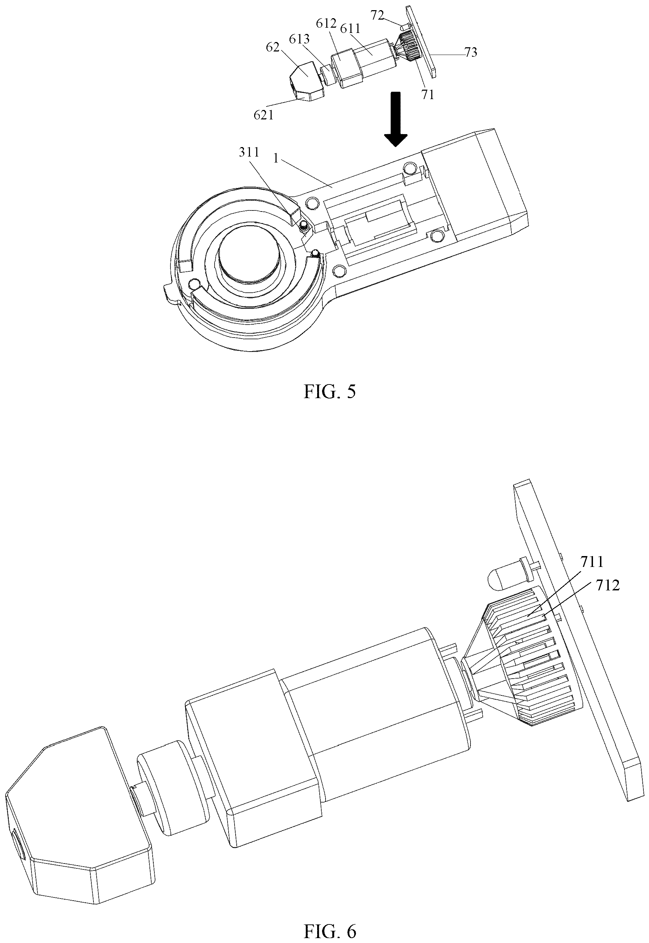

However, it should be noted that there are three methods of setting the acting surfaces of the first rotating frame 31 and the second rotating frame 32 (only one is emphatically illustrated in the present example in conjunction with the accompanying drawings):
1. The first rotating frame 31 is provided with a stress slope surface 311, and the telescopic locking portion is provided with a corresponding stress slope surface 311 at a position corresponding to the first rotating frame 31. In this way, the telescopic locking portion is pushed away from the first rotating frame 31 so that a friction force is generated between the first friction surface on the first rotating frame 31 and the second base 2 to realize self-locking.
2. Similarly, the second rotating frame 32 is provided with a stress slope surface, and the telescopic locking portion is provided with a corresponding stress slope surface at a position corresponding to the second rotating frame 32. In this way, the telescopic locking portion is pushed away from the second rotating frame 32 so that a friction force is generated between the first friction surface on the second rotating frame 32 and the second base 2 to realize self-locking.
3. Referring to FIG. 4 and as described above, both the first rotating frame 31 and the second rotating frame 32 are provided with stress slope surfaces, which can be symmetrically or asymmetrically corresponding. Taking FIG. 5 as an example, the first rotating frame and the second rotating frame form an equilateral inverted trapezoidal space. Specifically, the inverted trapezoidal space is formed between the stress slope surfaces of the first rotating frame 31 and the second rotating frame 32, the shape of the telescopic locking portion matches with the inverted trapezoidal space, and the inverted trapezoidal space formed by the first rotating frame 31 and the second rotating frame 32 can make the force more uniform.

Overall, the third method works best when implemented in concrete terms - the force applied during self-locking is uniform, and the first rotating frame 31 and the second rotating frame 32 are symmetrically and stably extended, but it does not mean that the first and second methods can't achieve the self-locking effect; although not accompanied by specific illustrations, but those skilled in the art should be able to draw the structure without a doubt.

It is also worth mentioning the forms of the first friction surface and the second friction surface, and the following three types are described in this example:
1. The first friction surface is formed by the outer wall surfaces of the first rotating frame 31 and the second rotating frame 32, the outer wall of which may be provided with a friction pattern or the like, and the second friction surface is likewise provided with a friction pattern of the inner wall;
2. The first friction surface and the second friction surface can be provided to be made of special materials, such as nylon products and rubber products, and are correspondingly attached to the first rotating frame 31, the second rotating frame 32 and the first base 1; and
3. The first friction surface is provided by the first friction member. Specifically, the outer side of the first rotating frame 31 and the outer side of the second rotating frame 32 are provided with slots, and two first friction members are mounted in corresponding slots of the first rotating frame 31 and the second rotating frame 32. Preferably, the first friction surface is selected as brake pad 33 by test; further, the second friction surface may be provided in the first or second of the above-described types.

In this embodiment, emphasis is placed on the third type, but it does not mean that the first and second types cannot implement and achieve the effect of friction self-locking. Although specific illustrations are not attached, those skilled in the art should be able to draw the structure without a doubt.

Further, the first base 1 is also provided with a chute which is used to cooperate with the telescopic locking portion to achieve telescoping in the sense of mechanical structure. The locking driving member is the power of the self-locking mechanism. Preferably, the locking driving member includes a driving motor 61, and the telescopic locking portion is a locking slider 62 disposed in the chute; the driving end of the driving motor 61 is provided with a threaded segment 614, the locking slider 62 is provided with a threaded groove, and the threaded segment 614 is inserted into the threaded groove. Specifically, the locking slider 62 cannot rotate axially along with the threaded segment 614 in the chute, so the threaded segment 614 acts as a lead screw here, and drives the locking slider 62 to reciprocate in the chute by the direction of self-rotation. The driving motor 61 is enabled to accurately adjust the rotation of the threaded segment 614 through a reduction gearbox 612, thereby accurately adjusting the advance and retreat of the locking slider 62, so that the whole self-locking process is accurate, smooth and natural.

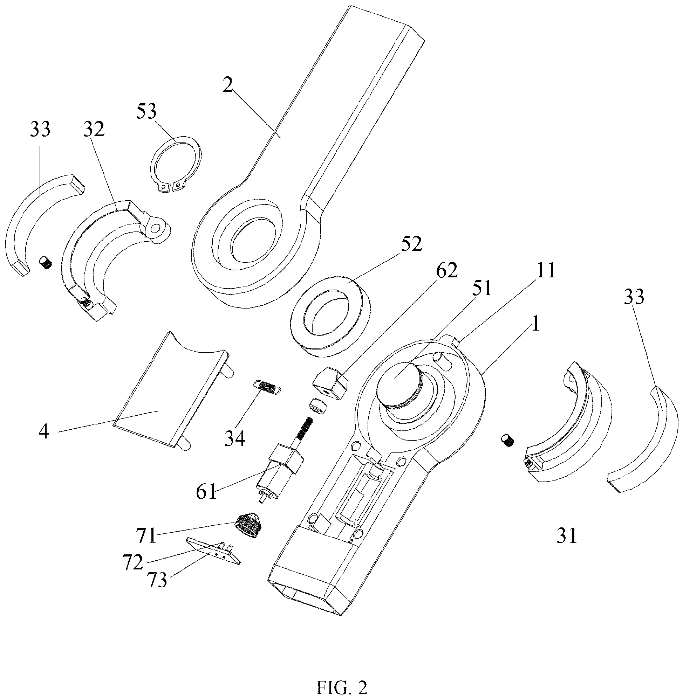

Human joints are often the joints of two limbs, which cooperate with the relative rotation of two limbs. As a preferred embodiment of the present example, referring to FIGS. 1, 2 and 3, the second base 2 is mounted on the first base 1 by a mounting mechanism, the mounting mechanism comprises a mounting groove, a bearing 52 and a rotating shaft 51. Specifically, the mounting groove is provided on the second base 2, the outer ring of the bearing 52 is fixedly mounted in the mounting groove; the rotating shaft 51 is disposed in the first compartment of the first base 1, and the inner ring of the bearing 52 is fixedly mounted on the rotating shaft 51. Preferably, the mounting groove may also be provided as a through groove described in FIG. 2, and then the clamp spring 53 is used for further fixed mounting. The structure facilitates the relative rotation of the first base 1 and the second base 2 without axial translocation, and the rotation is smooth and stable.

As a preferred embodiment of the present example, the free ends of the first rotating frame 31 and the second rotating frame 32 are connected by an elastic return member 34, which helps the first rotating frame and the second rotating frame to quickly disengage from the first base.

Further, the elastic return member 34 is preferably an extension spring, or an elastic resin or rubber can be selected. The purpose of the elastic return member is to reset and stretch the first rotating frame 31 and the second rotating frame 32, and everything that can achieve this purpose should be included in the selection range of the elastic return member.

It is worth mentioning that when the elastic return member 34 is not used, the joint rotation can be realized when the locking driving member is unlocked, but the addition of the elastic return member 34 makes the solution better.

As a preferred embodiment of the present example, referring to FIGS. 4, 5, 6 and 7, the driving motor 61 specifically comprises a miniature DC motor 611, a reduction gearbox 612, and a mounting bearing 613. The reduction gearbox 612 is mounted on the miniature DC motor 611, the position of the driving shaft is limited by the mounting bearing 613, and the entire driving motor 61 is mounted in the first base 1 in the form of mounting groove. The first base 1 is provided with groove whose shape and position are adapted to the driving motor 61 to fit the mounting limit. The driving motor is clamped in the form of groove in the prior art, so a redundant description is not made.

As a preferred embodiment of the present example, referring to FIG. 2, the present invention also includes an outer cover plate 4, which is mounted on the first base 1 and used to cover the driving motor 61 and other components. The outer cover plate 4 is disposed in a "post and slot" manner. The first base 1 is provided with a slot, and then the outer cover plate 4 is provided with a post, which is inserted into the slot for limiting, and then a bolt through hole connection or screw punch hole connection is made.

By adopting the above technical solution, the present invention has the following technical effects:
1. It abandons the structure of the motor group or hydraulic cylinder, the self-locking is realized by friction, the size of the friction depends on the pressure on the one hand and depends on friction coefficient on the other hand, so the power demand is not high, which can reduce the overall power consumption, prolong the useful time of the battery, improve the overall endurance, and also reduce the weight and cost of the whole machine.
2. The self-locking mechanism is widely used because of its compact structure and small size, which can be applied to the self-locking of the knee joint or other places, and is not limited to exoskeleton robots.

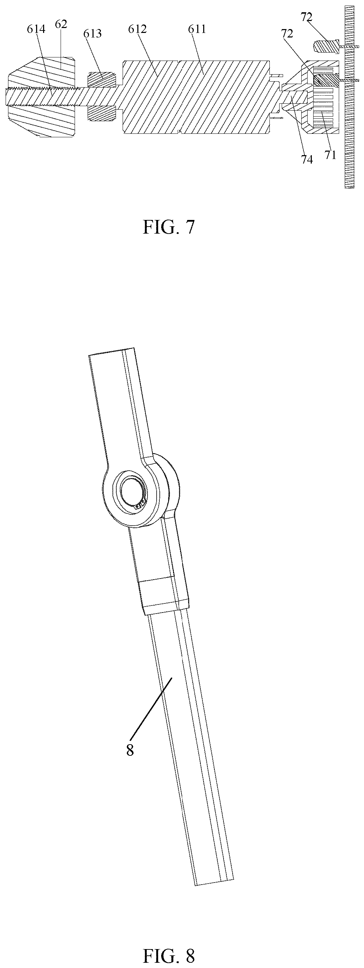

Referring to FIG. 8, the present invention also provides an exoskeleton knee joint, which comprises a shank connecting rod 8, a thigh connecting rod and the above exoskeleton joint self-locking mechanism. The shank connecting rod 8 is connected to the first base 1, and the thigh connecting rod is connected to the second base 2. The self-locking mechanism is disposed at the knee joint, which can start the locking driving member to complete the self-locking to assist standing when standing, and can unlock when walking to complete the activities at the knee joint.

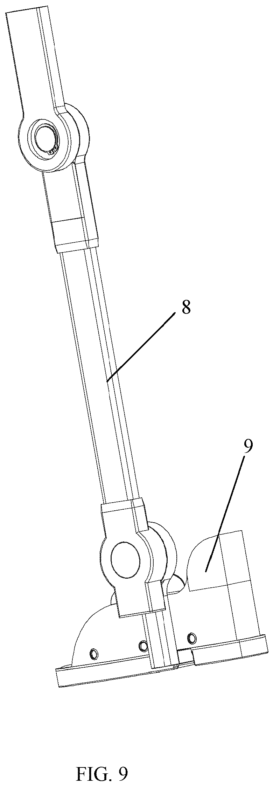

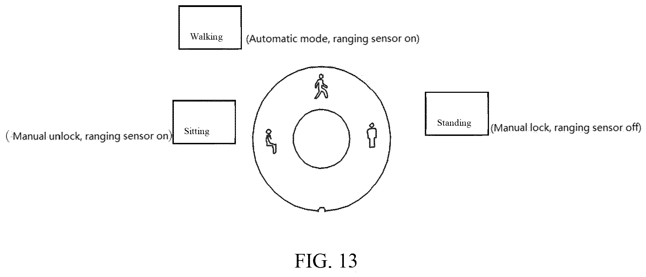

Referring to FIGS. 9-13, combined with FIGS. 1-5, the present invention also provides an exoskeleton bionic rehabilitation robot, which includes the above exoskeleton knee joint and an ankle-foot component 9, and the ankle-foot component 9 is connected to the shank connecting rod 8 of the exoskeleton knee joint. As mentioned above, the locking driving member can be started to complete self-locking to assist standing when standing, and the activities at the knee joint can be unlocked when walking.

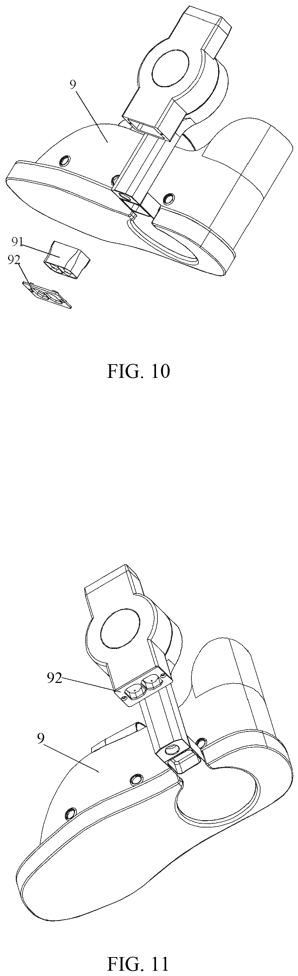

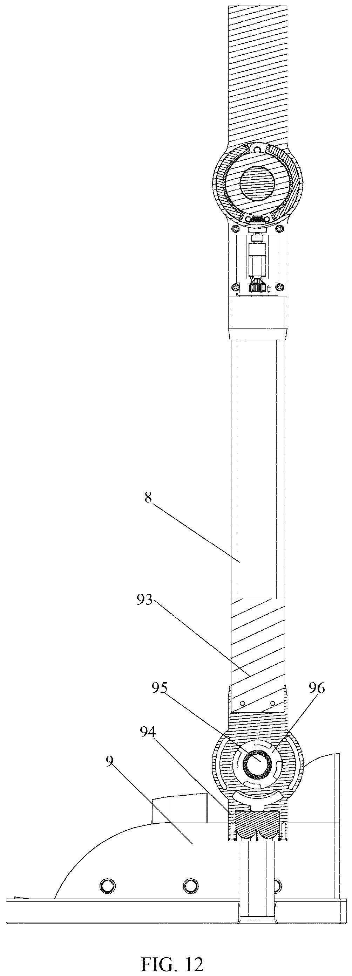

The following is described in detail by way of specific embodiments in conjunction with the accompanying drawings:
Referring to FIGS. 11 and 12, in order to improve the use experience of patients, as a preferred embodiment of this example, the present invention also includes a control unit, which comprises a control motherboard 73 and a ranging sensor 91, wherein the control motherboard 73 is in control connection to the locking driving member; the ranging sensor 91 is provided on the ankle-foot component 9 to measure a distance between the ankle-foot component 9 and the walking surface, and the ranging sensor 91 is in signal connection to the control motherboard 73. The distance between the foot and the walking surface (ground) is measured to determine whether the leg walks or stands. When standing, the distance between the ranging sensor 91 and the walking surface is the smallest, then self-locking is realized to assist standing; and when the distance between the ranging sensor 91 and the walking surface becomes larger, it is in the state of walking or preparing to walk, and activities of the knee joint are completely by the progressive unlocking.

As shown in FIG. 11, the ankle-foot component 9 and the shank connecting rod 8 are also rotationally connected, and the connection mode is similar to that of the first base 1 and the second base 2 in structure. Specifically, a third base 93 is disposed on the shank connecting rod 8, a fourth base 94 is disposed on the ankle-foot component 9, an ankle-foot rotating shaft 95 is disposed on the third base 93, an ankle-foot bearing 96 is disposed on the fourth base 94, and the fourth base 94 is mounted on the third base 93 through the ankle-foot component 9.

Preferably, the ranging sensor 91 is mounted at one end of the third base 93 facing the walking surface, and is mounted inside the third base 93, and then fixed with a ranging sensor bracket 92, which does not block the ranging sensing of the ranging sensor 91.

Further, the present invention also includes a remote control, which is provided with a basic function control module to communicate with the control motherboard 73. As an external manifestation and control form of a control unit, its technology is only the standard component of the preset program, and no redundant explanation will be made here; specifically, it implements the following functions:
Referring to FIGS. 11 and 12, when switching to a "sitting" mode, the ranging sensor 91 is turned off instantly, then the driving motor 61 is automatically reset and the locking slider 62 is retreated to the bottom end, under the action of the elastic return member 34 (extension spring), the first rotating frame 31 and the second rotating frame 32 are rotationally attached along a pivot shaft; at the same time, two brake pads 33 on the first rotating frame 31 and the second rotating frame 32 are driven to rotate and form a clearance fit with the inner wall of the second base 2 of the thigh connecting rod. At this time, the first base 1 and the second base 2 can be unlocked and rotated freely. But the first base 1 and the second base 2 are provided with rotation angle mechanical limits. The rotation angle mechanical limit means that when the joint stands at 180°, the first base 1 and the second base 2 are limited to each other by the angle limiting block 11 (the angle limiting block on the first base 1 is illustrated, the second base 2 is also provided with an angle limiting block without any illustration, but its position corresponds to that on the first base, so it can be undoubtedly concluded that the two angle limiting blocks 11 are abutted against each other when the joint stands at 180°), to ensure that the patient can only move within the ergonomic angle range and prevent the anti-joint rotation from bringing secondary injury to the patient. At this time, the patient can complete a sitting activity.

When the patient is lifted, it switches to a "standing" mode, the ranging sensor 91 is still not turned on, the driving motor 61 works rapidly to push the locking slider 62 to the maximum displacement of the top end, the first rotating frame 31 and the second rotating frame 32 are pushed by the locking slider 62 and quickly open, and at the same time, the two brake pads 33 squeeze and rub the inner wall of the second base 2 and lock the second base 2 and the rotating outward expanding locking member, thereby realizing the locking of the second base 2 and the first base 1, locking the rotational freedom and realizing the patient's assisted standing.

When switching to a "walking" mode, the ranging sensor 91 is activated for operation. Before standing and walking, the left and right knee joint self-locking mechanisms are locked and prohibited from rotating to support human body weight. At the beginning of walking, when the center of gravity of the human body moves to the right leg, the left thigh rotates and rises, which drives the shank and ankle joint to rise. The ranging sensor 91 detects a change of an initial distance value, feeds back and starts the driving motor 61 to work in time, and the locking slider 62 returns to drive the brake pad 33 to reset, thus unlocking the left knee joint to complete knee bending. Then, the patient slowly shifts the center of gravity forward to the left leg. During the falling of the left shank and ankle joint, the ranging sensor 91 detects that the distance value decreases and gradually recovers to the initial value. The driving motor 61 is started again to push the locking slider 62, and the friction force is slowly increased before the left foot lands until it is completely locked and supports the human body weight when landing.

The left leg's support and the right leg's walking share the same principle of action, so the cycle is repeated to complete the walking action and help the rehabilitation of the patient's lower limbs.

Further, the ranging sensor 91 includes but is not limited to an infrared ranging sensor 91, a laser ranging sensor 91, an ultrasonic ranging sensor 91 and a radar ranging sensor 91. The range sensor 91 is designed to measure the distance between the ankle-foot component and the walking surface to determine whether the leg is standing or lifting from the ground to prepare for walking.

Further, referring to FIGS. 6 and 7, the exoskeleton bionic rehabilitation robot also includes a locking measuring mechanism, which comprises a synchronous shaft 74, a signal transmitter, a signal receiver and a measuring turntable; wherein the synchronous shaft 74 rotates synchronously with the driving shaft of the driving motor 61; the signal transmitter and the signal receiver are disposed opposite to each other at intervals; the rotation center of the measuring turntable is connected to the synchronous shaft 74, and the edge spacing of the measuring turntable is cyclically provided with a signal marked area 711 and a signal unmasked area 712; and the measuring turntable rotates with the synchronous shaft 74 to circularly block/switch on the signal connection between the signal transmitter and the signal receiver. By synchronizing the driving shaft with the synchronous shaft 74, the measuring turntable also rotates, the number of times that the signal transmitter and signal receiver go through the signal marked area and a signal unmasked area till realizing the self-locking state is counted, so that an angle at which the measuring turntable has turned can be read and recorded, which can prevent the excessive rotation of the driving motor 61 from damaging other parts, and can also control the time to reach this angle, so that the speed of self-locking can be controlled, and the bionic real knee joint movement can be improved for comfort and safety.

As a preferred embodiment of the present example, the signal transmitter and the signal receiver are a set of infrared transceiver photodiode pairs 72. Further, the measuring turntable is a raster encoder 71.

To control the feed stroke of the slider and realize the adjustable and controllable friction force, the raster encoder 71 and the infrared transceiver photodiode pairs 72 are introduced for counting, which can give electrical signals when the contact is disconnected or connected through infrared communication. When the driving motor 61 drives the raster encoder 71 to rotate from the initial position, the infrared communication between the pairs and the raster encoder is blocked every certain rotation angle. By counting the number of times that the communication is blocked, the rotation angle of the synchronous shaft can be calculated. The rotation angle of the synchronous shaft needed to realize complete unlocking and locking of the knee joint can be obtained by calculation or test. When the rotation angle of the driving motor 61 reaches this value, a signal can be given by the control circuit to stop the rotation of the driving motor 61, to completely unlock and lock the knee joint. At the same time, by limiting the maximum rotation angle of the driving motor 61, damage to the motor and the mechanical structure can also be prevented. Since the reduction gearbox 612 is provided, the numerical difference between the rotation angle of the raster encoder 71 and the feed distance of the locking slider 62 can be amplified, and the control accuracy can be improved. On the other hand, since the unlocking-locking of the knee joint is controlled by the friction force between the brake pad 33 and the inner wall of the second base 2, it is possible to control the magnitude and change rate of this friction force by controlling the feed speed and the feed stroke of the locking slider 62, and to realize the switching between the unlocking, partial locking and locking states of the knee joint. A buffer state of partial locking is added between locking and unlocking states; in this state, the friction force is partially retained, the joint rotation rate is limited, the danger caused by sudden state change is eliminated, and the bionic real knee joint movement can be improved for comfort and safety.

Further, the driving motor 61 is a coaxial motor, and the synchronous shaft 74 and the driving shaft of the driving motor 61 are disposed to be coaxial but at different ends.

By adopting the above technical solution, the present invention has the following technical effects:
1. The present invention uses the motor to drive the locking slider to change the friction force between the brake pad and the friction surface to control the unlocking and locking of the knee joint. Compared with the prior art, which uses the friction force between the torsion spring and the friction surface to generate torque, the present invention is more reliable and durable, and is easier to maintain and replace in structure.
2. The present invention can control the change amount and speed of friction force between the brake pad and friction surface by controlling the rotation speed and rotation number of motor, to realize the function of gradually changing friction force and retaining partial friction force. However, in the prior art, the friction force changes sharply when the knee joint is opened and closed. Therefore, the present invention is more comfortable and safe for the wearer and has bionic characteristics.
3. In the prior art, the swing angle of the wearer's thigh is measured by an angle sensor to control the unlocking and locking of the knee joint. The present invention uses a ranging sensor to control the unlocking and locking of the knee joint by measuring the lifting or lowering of the ankle, which enlarges the measuring interval and is more sensitive to slight changes.
4. The present invention can manually control the unlocking and locking of the knee joint by means of remote control and the like, to protect the wearer in the process of sitting down, standing up and standing, save electric energy, and can also automatically control the knee joint to unlock and lock at different stages of walking during the wearer's walking, to imitate the state of the knee joint when human beings walk, which makes the exoskeleton movement more natural and comfortable.
5. The present invention adopts the way of controlling the friction force to lock and unlock the knee joint, so that the unlocking and locking of the knee joint are not completed instantaneously, but is gradually locked and unlocked with the increase and decrease of the friction force, which is more in line with the walking habits of the wearer and safer.
6. The present invention has the advantages of compact structure, small size, low energy consumption and prolonged endurance.

It should be noted that the "/" involved in the expression in the description is an optional meaning, which is interpreted as the meaning of "or". The structure, proportion, size, etc. shown in the drawings of this description are only used to cooperate with the contents disclosed in the description, for people familiar with this art to understand and read; it is not intended to limit the conditions under which the present invention can be implemented, so it has no technical significance. Any modification of structure, change of proportional relationship, or adjustment of size should still fall within the scope of the technical content disclosed by the present invention without affecting the effect and purpose achieved by the present invention. Meanwhile, the terms such as "upper", "lower", "left", "right", "middle" and "one" used in this description are for ease of description only, and are not intended to limit the practicable scope of the present invention. Changes or adjustments in their relative relations are also regarded as the practicable scope of the present invention without substantial changes in technical contents.

Various changes may be made to the present invention by ordinary technicians skilled in the art based on the above description. Thus, certain details in the embodiments are not intended to limit the present invention without departing from the scope of the claims of the present invention, and the present invention is to be protected within the scope defined in the appended claims.

## Claims

1. An exoskeleton joint self-locking mechanism, comprising
a first base (1) having a first compartment;
a rotating outward expanding locking member disposed in the first compartment, the rotating outward expanding locking member comprising a first rotating frame (31) and a second rotating frame (32), and outer sides of the first rotating frame (31) and the second rotating frame (32) having a first friction surface; and one end of the first rotating frame (31) being pivoted with one end of the second rotating frame (32), and other ends of the first rotating frame (31) and the second rotating frame (32) being relatively free ends;
a second base (2) having a second compartment; the second base (2) being rotationally mounted on the first base (1), and an inner wall of the second compartment enclosing the rotating outward expanding locking member and forming a second friction surface matching the first friction surface;
a locking driving member capable of applying/canceling a force pushing away from a free end of the first rotating frame (31) and a free end of the second rotating frame (32), to make the first friction surface close contact to lock/unlock the second friction surface; further, the free end of the first rotating frame (31) and/or the free end of the second rotating frame (32) being provided with a stress slope surface (311), the locking driving member being provided with a telescopic locking portion, the telescopic locking portion having a force applying slope surface (621), and the force applying slope surface (621) applying force to the stress slope surface (311) along with the telescopic locking portion, wherein the first base (1) includes a chute, the locking driving member includes a driving motor (61), and the telescopic locking portion is a locking slider (62) disposed in the chute; and
a locking measuring mechanism comprising:
a synchronous shaft (74) rotating synchronously with a driving shaft of the driving motor (61);
a signal transmitter and a signal receiver disposed opposite to each other at intervals;
a measuring turntable, a rotation center of the measuring turntable being connected to the synchronous shaft (74), and an edge spacing of the measuring turntable being cyclically provided with a signal marked area (711) and a signal unmasked area (712); and the measuring turntable rotating with the synchronous shaft (74) to circularly block/switch on a signal connection between the signal transmitter and the signal receiver.

2. The exoskeleton joint self-locking mechanism of claim 1, **characterized in that** an inverted trapezoidal space is formed between the stress slope surfaces (311) of the first rotating frame (31) and the second rotating frame (32), and a shape of the telescopic locking portion matches with the inverted trapezoidal space.

3. The exoskeleton joint self-locking mechanism of claim 1, **characterized in that** a driving end of the driving motor (61) is provided with a threaded segment (614), the locking slider (62) is provided with a threaded groove, and the threaded segment (614) is inserted into the threaded groove.

4. The exoskeleton joint self-locking mechanism of claim 1, **characterized in that** the second base (2) is mounted to the first base (1) through a mounting mechanism, and the mounting mechanism comprises:
a mounting groove provided on the second base (2);
a bearing (52), an outer ring of the bearing (52) being fixedly mounted in the mounting groove; and
a rotating shaft (51) disposed in the first compartment of the first base (1), and an inner ring of the bearing (52) being fixedly mounted on the rotating shaft (51).

5. The exoskeleton joint self-locking mechanism of claim 1, **characterized in that** the free end of the first rotating frame (31) and the free end of the second rotating frame (32) are connected by an elastic return member (34).

6. An exoskeleton knee joint, **characterized in** comprising a shank connecting rod (8), a thigh connecting rod and the exoskeleton joint self-locking mechanism of claim 3; and the shank connecting rod (8) is connected to the first base (1), and the thigh connecting rod is connected to the second base (2).

7. An exoskeleton bionic rehabilitation robot, **characterized in** comprising the exoskeleton knee joint of claim 6 and an ankle-foot component (9) which is connected to the shank connecting rod (8) of the exoskeleton knee joint.

8. The exoskeleton bionic rehabilitation robot of claim 7, **characterized in that** the exoskeleton bionic rehabilitation robot further comprises a control unit comprising:
a control motherboard (73) being in control connection to the locking driving member; and
a ranging sensor (91) disposed on the ankle-foot component (9) to measure a distance between the ankle-foot component (9) and a walking surface, and the ranging sensor (91) being in signal connection to the control motherboard (73).

9. The exoskeleton bionic rehabilitation robot of claim 8, **characterized in that** the ranging sensor (91) includes an infrared ranging sensor, a laser ranging sensor, an ultrasonic ranging sensor, and a radar ranging sensor.

10. The exoskeleton bionic rehabilitation robot of claim 8, **characterized in that** the signal transmitter and the signal receiver are a set of infrared transceiver photodiode pairs (72).

11. The exoskeleton bionic rehabilitation robot of claim 10, **characterized in that** the measuring turntable is a raster encoder (71).

12. The exoskeleton bionic rehabilitation robot of claim 8, **characterized in that** the driving motor (61) is a coaxial motor, and the synchronous shaft (74) and the driving shaft of the driving motor (61) are disposed to be coaxial but at different ends.

## Patentansprüche

1. Selbstsperrender Mechanismus für ein Exoskelettgelenk, umfassend:
eine erste Basis (1) mit einer ersten Kammer;
ein rotierendes, sich nach außen ausdehnendes Verriegelungselement, das in der ersten Kammer angeordnet ist, wobei das rotierende, sich nach außen ausdehnende Verriegelungselement einen ersten rotierenden Rahmen (31) und einen zweiten rotierenden Rahmen (32) umfasst und die Außenseiten des ersten rotierenden Rahmens (31) und des zweiten rotierenden Rahmens (32) eine erste Reibungsfläche aufweisen; und wobei ein Ende des ersten rotierenden Rahmens (31) mit einem Ende des zweiten rotierenden Rahmens (32) gelenkig verbunden ist und die anderen Enden des ersten rotierenden Rahmens (31) und des zweiten rotierenden Rahmens (32) relativ freie Enden sind;
eine zweite Basis (2) mit einer zweiten Kammer, wobei die zweite Basis (2) drehbar an der ersten Basis (1) angebracht ist und eine Innenwand der zweiten Kammer das rotierendes, sich nach außen ausdehnende Verriegelungselement umschließt und eine zweite Reibungsfläche bildet, die mit der ersten Reibungsfläche abgestimmt;
ein Verriegelungsantriebselement, das eine Kraft aufbringen/aufheben kann, die von einem freien Ende des ersten rotierenden Rahmens (31) und einem freien Ende des zweiten rotierenden Rahmens (32) wegdrückt, um die erste Reibungsfläche in engen Kontakt zu bringen, um die zweite Reibungsfläche zu verriegeln/entriegeln; wobei ferner das freie Ende des ersten rotierenden Rahmens (31) und/oder das freie Ende des zweiten rotierenden Rahmens (32) mit einer Spannungsschrägfläche (311) versehen ist, wobei das Verriegelungsantriebselement mit einem teleskopischen Verriegelungsabschnitt versehen ist, wobei der teleskopische Verriegelungsabschnitt eine kraftaufbringende Schrägfläche (621) aufweist, und die kraftaufbringende Schrägfläche (621) zusammen mit dem teleskopischen Verriegelungsabschnitt eine Kraft auf die Spannungsschrägfläche (311) ausübt, wobei die erste Basis (1) eine Rutsche umfasst, das Verriegelungsantriebselement einen Antriebsmotor (61) umfasst und der teleskopische Verriegelungsabschnitt ein Verriegelungsschieber (62) ist, der in der Rutsche angeordnet ist; und
einen Verriegelungsmessmechanismus, umfassend:
eine Synchronwelle (74), die sich synchron mit einer Antriebswelle des Antriebsmotors (61) dreht;
einen Signalsender und einem Signalempfänger, die einander in Abständen gegenüberliegen;
einen Messdrehteller, wobei ein Drehpunkt des Messdrehtellers mit der Synchronwelle (74) verbunden ist und ein Kanten des Messdrehtellers beabstandet zyklisch mit einem signalmarkierten Bereich (711) und einem signalunmarkierten Bereich (712) versehen ist; Wobei der Messdrehtisch sich mit der Synchronwelle (74) dreht, um eine Signalverbindung zwischen dem Signalsender und dem Signalempfänger kreisförmig zu blockieren/umzuschalten.

2. Selbstsperrender Mechanismus für ein Exoskelettgelenk nach Anspruch 1, **dadurch gekennzeichnet, dass** ein umgekehrter trapezförmiger Raum zwischen den Spannungsschrägflächen (311) des ersten rotierenden Rahmens (31) und des zweiten rotierenden Rahmens (32) gebildet wird, und dass eine Form des teleskopischen Verriegelungsabschnitts mit dem umgekehrten trapezförmigen Raum übereinstimmt.

3. Selbstsperrender Mechanismus für ein Exoskelettgelenk nach Anspruch 1, **dadurch gekennzeichnet, dass** ein Antriebsende des Antriebsmotors (61) mit einem Gewindesegment (614) versehen ist, der Verriegelungsschieber (62) mit einer Gewindenut versehen ist und das Gewindesegment (614) in die Gewindenut eingesetzt ist.

4. Selbstsperrender Mechanismus für ein Exoskelettgelenk nach Anspruch 1, **dadurch gekennzeichnet, dass** die zweite Basis (2) an der ersten Basis (1) durch einen Befestigungsmechanismus befestigt ist, und der Befestigungsmechanismus umfasst:
eine Montagenut, die an der zweiten Basis (2) vorgesehen ist;
ein Lager (52), wobei ein Außenring des Lagers (52) fest in der Montagenut montiert ist; und
eine Drehwelle (51), die in der ersten Kammer der ersten Basis (1) angeordnet ist und ein Innenring des Lagers (52) fest auf der Drehwelle (51) montiert ist.

5. Selbstsperrender Mechanismus für ein Exoskelettgelenk nach Anspruch 1, **dadurch gekennzeichnet, dass** das freie Ende des ersten rotierenden Rahmens (31) und das freie Ende des zweiten rotierenden Rahmens (32) durch ein elastisches Rückstellelement (34) verbunden sind.

6. Exoskelett-Kniegelenk, **dadurch gekennzeichnet, dass** es eine Unterschenkel-Verbindungsstange (8), eine Oberschenkel-Verbindungsstange und den Selbstsperrender Mechanismus für ein Exoskelettgelenk nach Anspruch 3 umfasst; und die Unterschenkel-Verbindungsstange (8) mit der ersten Basis (1) verbunden ist und die Oberschenkel-Verbindungsstange mit der zweiten Basis (2) verbunden ist.

7. Bionischer Exoskelett-Rehabilitationsroboter, **dadurch gekennzeichnet, dass** er das Exoskelett-Kniegelenk nach Anspruch 6 und eine Knöchel-Fuß-Komponente (9) umfasst, die mit der Unterschenkel-Verbindungsstange (8) des Exoskelett-Kniegelenks verbunden ist.

8. Bionischer Exoskelett-Rehabilitationsroboter nach Anspruch 7, **dadurch gekennzeichnet, dass** der bionische Exoskelett-Rehabilitationsroboter außerdem eine Steuereinheit umfasst, wobei die Steuereinheit umfasst:
eine Steuerplatine (73), die in Steuerverbindung mit dem Verriegelungsantriebselement steht; und
einen Entfernungssensor (91), der an der Knöchel-Fuß-Komponente (9) angeordnet ist, um einen Abstand zwischen der Knöchel-Fuß-Komponente (9) und einer Gehoberfläche zu messen, und wobei der Entfernungssensor (91) in Signalverbindung mit der Steuerplatine (73) steht.

9. Bionischer Exoskelett-Rehabilitationsroboter nach Anspruch 8, **dadurch gekennzeichnet, dass** der Entfernungssensor (91) einen Infrarot-Entfernungssensor, einen Laser-Entfernungssensor, einen Ultraschall-Entfernungssensor und einen Radar-Entfernungssensor umfasst.

10. Bionischer Exoskelett-Rehabilitationsroboter nach Anspruch 8, **dadurch gekennzeichnet, dass** der Signalsender und der Signalempfänger ein Satz von Infrarot-Transceiver-Photodiodenpaaren (72) sind.

11. Bionischer Exoskelett-Rehabilitationsroboter nach Anspruch 10, **dadurch gekennzeichnet, dass** der Messdrehtisch ein Raster-Encoder (71) ist.

12. Bionischer Exoskelett-Rehabilitationsroboter nach Anspruch 8, **dadurch gekennzeichnet, dass** der Antriebsmotor (61) ein Koaxialmotor ist und die Synchronwelle (74) und die Antriebswelle des Antriebsmotors (61) koaxial, aber an unterschiedlichen Enden angeordnet sind.

## Revendications

1. Mécanisme d'articulation d'exosquelette à verrouillage automatique, **caractérisé en ce qu'**il comprend :
une première base (1) qui a un premier compartiment ;
un élément de verrouillage rotatif en expansion vers l'extérieure disposé dans ledit premier compartiment, qui inclut une première charpente rotative (31) et une deuxième charpente rotative (32) dont des côtés extérieurs ont une première surface de friction, et dont les uns des extrémités se lient en pivot l'une à l'autre, et dont les autres des extrémités sont des extrémités relativement libres ;
une deuxième base (2) qui est disposée en rotation sur ladite première base (1) et qui a un second compartiment dont une paroi intérieure entoure ledit élément de verrouillage rotatif en expansion vers l'extérieure et forme une deuxième surface de friction correspondant à ladite première surface de friction;
un élément d'entraînement de verrouillage, qui est capable d'appliquer/de retirer une force poussant loin d'une extrémité libre de ladite première charpente rotative (31) et une extrémité libre de ladite deuxième charpente rotative (32) dont l'une et/ou les deux dispose en outre d'une surface de pente à contrainte (311), afin de mettre ladite première surface de friction en contact étroit pour verrouiller/déverrouiller ladite deuxième surface de friction, et qui dispose d'une partie de verrouillage télescopique qui a une surface de pente à appliquer une force (621) qui applique une force à ladite surface de pente de contrainte (311) avec ladite partie de verrouillage télescopique, où ladite première base (1) inclut une glissière, ledit élément d'entraînement de verrouillage inclut un moteur d'entraînement (61), et ladite partie de verrouillage télescopique est un glissoir de verrouillage (62) disposé dans ladite glissière; et
un mécanisme de mesure de verrouillage qui inclut :
un arbre synchrone (74) qui tourne synchroniquement avec un arbre d'entraînement dudit moteur d'entraînement (61) ;
un émetteur de signal et un récepteur de signal disposés l'un en face de l'autre à intervalles réguliers; et
un plateau tournant de mesure, dont un centre de rotation est lié audit arbre synchrone (74), et dont un espacement de bord dispose cycliquement d'une zone marquée de signal (711) et d'une zone démasquée de signal (712), et qui tourne avec ledit arbre synchrone (74) afin de mettre en arrêt / mettre en marche circulairement une connexion de signal entre ledit émetteur de signal et ledit récepteur de signal.

2. Mécanisme d'articulation d'exosquelette à verrouillage automatique selon la revendication 1, **caractérisé en ce qu'**un espace trapézoïdal inversé est formé entre les surfaces de pente de contrainte (311) de ladite première charpente rotative (31) et ladite deuxième charpente rotative (32), et une forme de ladite partie de verrouillage télescopique correspond à l'espace trapézoïdal inversé.

3. Mécanisme d'articulation d'exosquelette à verrouillage automatique selon la revendication 1, **caractérisé en ce qu'**une extrémité d'entraînement dudit moteur d'entraînement (61) dispose d'un segment à pas de vis (614), ledit glissoir de verrouillage (62) dispose d'une rainure à pas de vis, et ledit segment à pas de vis (614) est inséré dans ladite rainure à pas de vis.

4. Mécanisme d'articulation d'exosquelette à verrouillage automatique selon la revendication 1, **caractérisé en ce que** ladite deuxième base (2) est disposée à ladite première base (1) au moyen d'un mécanisme d'installation qui inclut :
une rainure d'installation disposée sur ladite deuxième base (2) ;
un roulement (52) dont un anneau extérieur est disposé de manière fixe dans ladite rainure d'installation; et
un arbre rotatif (51) qui est disposé dans ledit premier compartiment de ladite première base (1), et sur lequel un anneau intérieur dudit roulement (52) est disposé de manière fixe.

5. Mécanisme d'articulation d'exosquelette à verrouillage automatique selon la revendication 1, **caractérisé en ce que** l'extrémité libre de ladite première charpente rotative (31) et l'extrémité libre de ladite deuxième charpente rotative (32) sont liées l'une à l'autre par un élément de retour élastique (34).

6. Articulation de genou d'exosquelette, **caractérisée en ce qu'**elle comprend une tige de liaison à jambe (8), une tige de liaison à cuisse et le mécanisme d'articulation d'exosquelette à verrouillage automatique selon la revendication 3 ; dans lesquels ladite tige de liaison à jambe (8) est liée à ladite première base (1), et ladite tige de liaison à cuisse est liée à ladite deuxième base (2).

7. Robot de rééducation d'exosquelette bionique, **caractérisé en ce qu'**il comprend l'articulation de genou d'exosquelette selon la revendication 6 et un assemblage de cheville-pied (9) qui est lié à la tige de liaison à jambe (8) de l'articulation de genou d'exosquelette.

8. Robot de rééducation d'exosquelette bionique selon la revendication 7, **caractérisé en ce qu'**il comprend en outre :
une carte mère de contrôle (73) en connexion de contrôle avec ledit élément d'entraînement de verrouillage; et
un capteur de télémétrie (91) qui est disposé sur ledit assemblage de cheville-pied (9) pour mesurer une distance entre ledit assemblage de cheville-pied (9) et une surface de marche, et qui est en connexion de signal avec ladite carte mère de contrôle (73).

9. Robot de rééducation d'exosquelette bionique selon la revendication 8, **caractérisé en ce que** ledit capteur de télémétrie (91) inclut un capteur de télémétrie infrarouge, un capteur de télémétrie laser, un capteur de télémétrie ultrasonique et un capteur de télémétrie radar.

10. Robot de rééducation d'exosquelette bionique selon la revendication 8, **caractérisé en ce que** ledit émetteur de signal et ledit récepteur de signal sont un groupe de paires de photodiodes d'émetteur-récepteur infrarouge (72).

11. Robot de rééducation d'exosquelette bionique selon la revendication 10, **caractérisé en ce que** ledit plateau tournant de mesure est un encodeur de trame (71).

12. Robot de rééducation d'exosquelette bionique selon la revendication 8, **caractérisé en ce que** ledit moteur d'entraînement (61) est un moteur coaxial, et ledit arbre synchrone (74) et l'arbre d'entraînement dudit moteur d'entraînement (61) sont disposés de manière coaxiale mais à des extrémités différentes.
